# EUROPÄISCHE PATENTANMELDUNG

(11) **EP 1 972 313 A1**
(43) Veröffentlichungstag der Anmeldung: **24.09.2008**
(21) Anmeldenummer: 07405094.9
(22) Anmeldetag: 20.03.2007
(51) Int. Cl.: A61F 9/01, A61F 9/011

(54) **Ophthalmologische Vorrichtung für die refraktive Korrektur eines Auges**

(71) Anmelder: SIE AG, Surgical Instrument Engineering, 2562 Port (CH)
(72) Erfinder: Rathjen, Christian, 28197 Bremen (DE)
(74) Vertreter: Vogel, Dany

(57) **Zusammenfassung**

Eine ophthalmologische Vorrichtung (1) für die refraktive Korrektur eines Auges (2), umfasst eine erste Laserquelle (Q1) zur Erzeugung eines gepulsten ersten Laserstrahls mit Femtosekundenlaserpulsen und eine zweite Laserquelle (Q2) zur Erzeugung eines zweiten Laserstrahls (L2) im mittelwelligen Infrarotbereich. Die ophthalmologische Vorrichtung (1) umfasst zudem einen gemeinsamen optischen Lichtprojektor (11) zur fokussierten Projektion des ersten Laserstrahls in einem ersten Betriebsmodus und zur fokussierten Projektion des zweiten- Laserstrahls (L2) in einem zweiten Betriebsmodus jeweils zum Auflösen von Augengewebe. Die Verwendung eines gemeinsamen optischen Lichtprojektors (11) zum Schneiden des Gewebelappens (22) mittels der Femtosekundenlaserpulse und zum nachfolgenden refraktiv korrigierenden Gewebeabtragen mittels des Laserstrahls (L2) im mittelwelligen IR Bereich hat den Vorteil, dass weder der Lichtprojektor (11) ausgewechselt noch der Patient umpositioniert werden muss, so dass dadurch weder die Genauigkeit noch die zeitliche Dauer des chirurgischen Eingriffs beeinträchtigt respektive verzögert werden.

## Beschreibung

### Technisches Gebiet

Die vorliegende Erfindung betrifft eine ophthalmologische Vorrichtung für die refraktive Korrektur eines Auges. Die Erfindung betrifft insbesondere eine ophthalmologische Vorrichtung für die refraktive Korrektur eines Auges, welche eine Laserquelle zur Erzeugung eines gepulsten Laserstrahls mit Femtosekundenlaserpulsen umfasst.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem Excimerlaser abgetragen wird, nachdem zuvor ein Hornhautlappen teilweise abgetrennt und weggeklappt wird. Solche Hornhautlappen werden mit mechanischen Mikrokeratomen oder mittels stark fokussierter Femtosekundenlaserpulsen geschnitten. Geeignete Femtosekundenlasersysteme erzeugen Laserpulse mit Pulsbreiten von typisch 100fs bis 1000fs (1fs=10⁻¹⁵s).

In der Patentanmeldung WO 03/057100 wird eine Vorrichtung für die refraktive Laserchirurgie beschrieben, welche ein Lasersystem zur Erzeugung eines gepulsten Laserstrahls mit Femtosekundenlaserpulsen und ein weiteres Lasersystem zur Erzeugung eines Laserstrahls im ultravioletten (UV) Bereich umfasst. Vorzugsweise werden beide Lasersysteme durch eine gemeinsame Pumplaserquelle im Infrarotbereich gespeist, wobei die UV Laserstrahlen mittels Frequenzvervielfachung aus dem Infrarotlicht erzeugt werden. In einer Ausführungsvariante wird zur Erzeugung des UV Laserstrahls ein Excimerlaser verwendet. Die Lichtstrahlen beider Lasersysteme werden einem gemeinsamen Scanner zugeführt, der sowohl zur Ablenkung der Femtosekundenlaserpulse beim Schneiden des Hornhautlappens als auch zur Ablenkung des UV Laserstrahls bei der refraktiven Korrektur der Augenhornhaut verwendet wird. Um sowohl Laserstrahlen im Infrarotbereich als auch Laserstrahlen im UV Bereich fokussiert in, respektive auf, das Augengewebe zu projizieren, müssen unterschiedliche optische Lichtprojektoren bereitgestellt werden, welche für die ausreichende Transmission von Infrarotlicht respektive UV Licht ausgestaltet sind. Geeignete Materialien, die sowohl Infrarotlicht als auch UV Licht ausreichend gut transmittieren sind zurzeit nicht bekannt. Mehrere optische Lichtprojektoren bedingen, dass die Lichtprojektoren entweder mechanisch ausgetauscht, positioniert und justiert werden müssen, oder dass der Patient von einem zum anderen Lichtprojektor bewegt werden muss. In beiden Fällen muss mit einer Beeinträchtigung der Genauigkeit des chirurgischen Eingriffs oder zumindest mit einem Unterbruch und einer zeitlichen Verlängerung des Eingriffs gerechnet werden.

### Darstellung der Erfindung

Es ist eine Aufgabe der vorliegenden Erfindung, eine neue ophthalmologische Vorrichtung für die refraktive Korrektur eines Auges mittels Laserpulsen vorzuschlagen, welche nicht die angeführten Nachteile des Stands der Technik aufweist. Es ist insbesondere eine Aufgabe der vorliegenden Erfindung, eine neue ophthalmologische Vorrichtung vorzuschlagen, welche eingerichtet ist, Laserstrahlen sowohl zum Schneiden eines Gewebelappens (Hornhautlappen) als auch zum refraktiv korrigierenden Gewebeabtrag mit einem gemeinsamen optischen Lichtprojektor fokussiert in respektive auf das Augengewebe zu projizieren.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass die ophthalmologische Vorrichtung für die refraktive Korrektur eines Auges, welche eine erste Laserquelle umfasst zur Erzeugung eines gepulsten ersten Laserstrahls mit Femtosekundenlaserpulsen, überdies eine zweite Laserquelle zur Erzeugung eines zweiten Laserstrahls im mittelwelligen Infrarotbereich umfasst. Zudem umfasst die ophthalmologische Vorrichtung einen gemeinsamen optischen Lichtprojektor zur fokussierten Projektion des gepulsten ersten Laserstrahls in einem ersten Betriebsmodus und zur fokussierten Projektion des zweiten Laserstrahls in einem zweiten Betriebsmodus jeweils zum Auflösen von Augengewebe (Augenhomhaut). Um das Augengewebe aufzulösen, ist der gemeinsame optische Lichtprojektor eingerichtet, den ersten Laserstrahl im ersten Betriebsmodus auf einen Brennpunkt im Innern des Augengewebes zu projizieren, und den zweiten Laserstrahl im zweiten Betriebsmodus auf einen Brennpunkt auf dem Augengewebe zu projizieren. Die erste Laserquelle zur Erzeugung der Femtosekundenlaserpulse arbeitet im IR Bereich (Infrarotlaser), beispielsweise bei einer Wellenlänge im Bereich von 1000nm bis 1200nm. Die zweite Laserquelle arbeitet im mittelwelligen IR Bereich, vorzugsweise im Bereich von 2.9µm bis 3.0µm (2900nm bis 3000nm). Dadurch, dass für den refraktiv korrigierenden Gewebeabtrag, anstelle eines herkömmlichen im UV Bereich operierenden Excimerlasers eine im mittelwelligen IR Bereich arbeitende Laserquelle eingesetzt wird, kann der selbe optische Lichtprojektor verwendet wurden, um sowohl die Femtosekundenlaserpulse zum Schneiden eines Gewebelappens (Hornhaulappen) als auch den im mittelwelligen IR Bereich liegenden Laserstrahl für den Gewebeabtrag fokussiert in, respektive auf, das Augengewebe zu projizieren. Die Verwendung eines gemeinsamen optischen Lichtprojektors zum Schneiden des Gewebelappens mittels der Femtosekundenlaserpulse und zum nachfolgenden refraktiv korrigierenden Gewebeabtragen mittels des Laserstrahls im mittelwelligen IR Bereich hat den Vorteil, dass weder Lichtprojektoren ausgewechselt werden müssen noch der Patient umpositioniert werden muss, so dass dadurch weder die Genauigkeit noch die zeitliche Dauer des chirurgischen Eingriffs beeinträchtigt respektive verzögert werden.

Vorzugsweise umfasst die ophthalmologische Vorrichtung Fokussiermittel, weiche eingerichtete sind zum Fokussieren der- Projektion des ersten Laserstrahls im ersten Betriebsmodus mit einem ersten Fokusdurchmesser und zum Fokussieren der Projektion des zweiten Laserstrahls im zweiten Betriebsmodus mit einem hinsichtlich des ersten Fokusdurchmessers um ein Mehrfaches grösseren zweiten Fokusdurchmesser. Der erste Fokusdurchmesser liegt beispielsweise im Bereich von 0.5µm bis 10µm, wohingegen der zweite Fokusdurchmesser im Bereich von 100µm bis 500µm liegt. Ein kleiner Fokusdurchmesser für die Projektion der Femtosekundenlaserpulse ermöglicht präzise, feine Schnitte im Innern des Augengewebes beim Schneiden des Gewebelappens. Der grössere Fokusdurchmesser ermöglicht ein grossräumigeres Abtragen von Hornhautgewebe bei weggeklapptem Gewebelappen.

In einer Ausführungsvariante umfasst die ophthalmologische Vorrichtung Mittel, welche eingerichtet sind, den ersten Laserstrahl in einem dritten Betriebsmodus mit einer hinsichtlich der Projektion des ersten Laserstrahls im ersten Betriebsmodus und hinsichtlich der Projektion des zweiten Laserstrahls im zweiten Betriebsmodus derart reduzierten Intensität in das Augengewebe zu projizieren, dass kein Augengewebe aufgelöst wird. Das heisst im dritten Betriebsmodus wird der erste Laserstrahl mit reduzierter Intensität in das Augengewebe eingestrahlt, so dass beispielsweise die Augenhornhaut durch das Licht im kurzwelligen IR Bereich des Femtosekundenlasers ausgeleuchtet wird, ohne dass das Augengewebe, insbesondere die Augenhornhaut, beschädigt wird. Dies ist insbesondere als vorbereitender Schritt zum Verfestigen der Augenhornhaut geeignet, wenn diese vorher mit Riboflavin (Vitamin B2) als Photosensibilisator behandelt wurde, und durch die Bestrahlung mit dem mittelwelligen IR Licht im Hornhautgewebe Quervernetzungen gebildet werden, die die Hornhaut festigen. Diese Verfestigen der Hornhaut kann beispielsweise in Kombination mit einem formgebenden Kontaktkörper (z.B. ein Applanationskörper) vorgenommen werden.

Vorzugsweise umfasst die ophthalmologische Vorrichtung ein Tiefenpositionierungsmodul zum Positionieren des optischen Lichtprojektors im ersten Betriebsmodus mit einem ersten Arbeitsabstand zu einer Bearbeitungsfläche im Auge und zum Positionieren des optischen Lichtprojektors im zweiten Betriebsmodus mit einem zweiten Arbeitsabstand zu der Bearbeitungsfläche. Die Fokussiermittel sind zudem eingerichtet, die Projektion des ersten Laserstrahls im ersten Betriebsmodus zum Schneiden des Gewebelappens auf die Bearbeitungsfläche zu fokussieren, und die Projektion des zweiten Laserstrahls im zweiten Betriebsmodus zum Abtragen von Augengewebe bei weggeklapptem Gewebelappen auf die Bearbeitungsfläche zu fokussieren. In einer Ausführungsvariante wird die Projektion des zweiten Laserstrahls im zweiten Betriebsmodus zum Abtragen von Augengewebe auf eine Bearbeitungsfläche fokussiert, die äquidistant ist zur Bearbeitungsfläche, die zum Schneiden des Gewebelappens verwendet wurde. Die Einstellung unterschiedlicher Arbeitsabstände beim Schneiden des Gewebelappens und beim refraktiv korrigierenden Abtragen des Homhautgewebes sowie die (automatisch) entsprechend angepasste Fokussierung der Laserstrahlen ermöglichen einerseits eine präzisere Behandlung durch die Wahl geeignet kurzer Arbeitsabstände für das Schneiden des Gewebelappens sowie andererseits eine Anpassung (Reduktion) der Laserenergie an kürzere Arbeitsabstände.

Vorzugsweise ist die erste Laserquelle eingerichtet ist, den ersten Laserstrahl mit einer Pulsrate zu erzeugen, die ein Mehrfaches der Pulsrate ist, welche die zweite Laserquelle für den zweiten Laserstrahl erzeugt. Die Pulsrate der Femtosekundenlaserpulse ist beispielsweise im Bereich von 10KHz bis 500KHz, wohingegen die Pulsrate beim im mittelwelligen IR Bereich liegenden zweiten Laserstrahl beispielsweise im Bereich von 50Hz bis 2KHz liegt. Durch die der zweiten Laserquelle eigene geringe Pulsrate ist es möglich bei der Gewebeabtragung durch den zweiten Laserstrahl Augenbewegungen beim Positionieren des Fokus einzelner Laserpulse zu berücksichtigten.

Die ophthalmologische Vorrichtung umfasst vorzugsweise ein Scannersystem zur Fokusbewegung bei der fokussierten Projektion des ersten Laserstrahls und bei der fokussierten Projektion des zweiten Laserstrahls. Zum Schneiden des Gewebelappens mittels der Femtosekundenlaserpulse wird der Fokus gemäss einem entsprechenden Abtastmuster bewegt; zur refraktiv korrigierenden Gewebeabtragung mittels des mittelwelligen IR Laserstrahls wird der Fokus bei weggeklapptem Gewebelappen gemäss einem geeigneten Algorithmus bewegt, wobei Augenbewegungen, die durch einen so genannten Eyetracker erfasst werden, vorzugsweise mitberücksichtigt werden.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Vorrichtung beim Schneiden eines Gewebelappens von der Augenhornhaut illustriert.
Figur 2 zeigt ein Blockdiagramm, welches schematisch eine ophthalmologische Vorrichtung beim Abtragen von Augengewebe illustriert.
Figur 3 zeigt ein Flussdiagram, welches den Ablauf beim Schneiden eines Gewebelappens und beim Abtragen von Augengewebe zur refraktiven Korrektur des Auges illustriert.

### Wege zur Ausführung der Erfindung

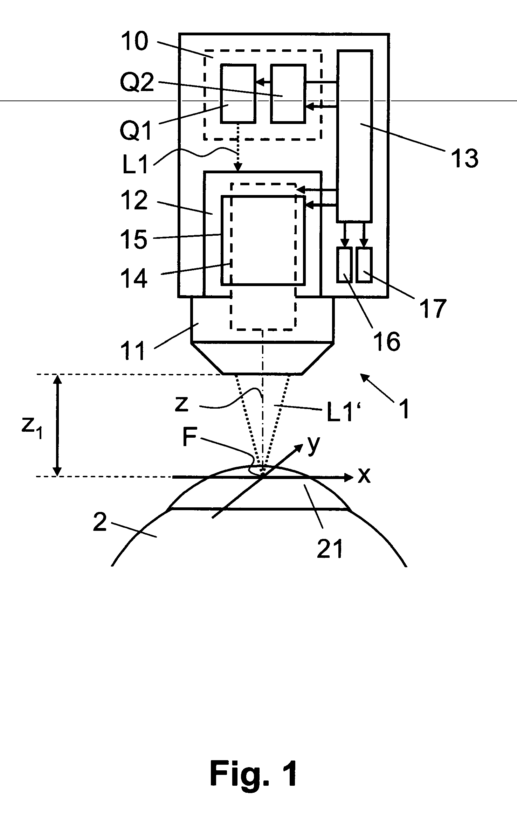

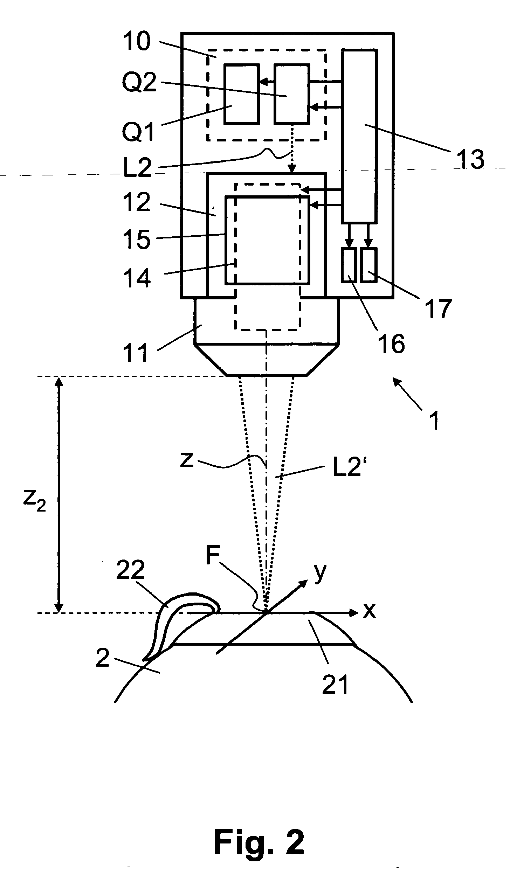

In den Figuren 1 und 2 bezeichnet das Bezugszeichen 1 eine ophthalmologische Vorrichtung, respektive eine ophthalmologische Vorrichtungsanordnung, mit zwei Laserquellen Q1, Q2 und einem mit den Laserquellen Q1, Q2 über ein optisches Übertragungssystem 12 verbundenen optischen Lichtprojektor 11 zur Erzeugung und fokussierten Projektion eines gepulsten Laserstrahls L1', L2' für die punktuelle Gewebeauflösung in einem Fokus F (Brennpunkt) im respektive auf dem Augengewebe, beispielsweise in oder auf der Augenhornhaut 21 (Cornea).

Zum besseren Verständnis soll hier angeführt werden, dass die Figuren 1 und 2 die ophthalmologische Vorrichtung 1 schematisch und vereinfacht darstellen. Zum Beispiel ist in den Figuren 1, 2 nicht präzise wiedergegeben, dass der Lichtprojektor 11 eine hohe (und regulierbare) numerische Apertur von mindestens 0.3 und vorzugsweise mehr als 0.4 aufweist, dass die ophthalmologische Vorrichtung 1 optional einen Saugring zur Befestigung am Auge 2 aufweist, oder dass die ophthalmologische Vorrichtung 1 optional einen Kontaktkörper (z.B. einen Applanationskörper) zur kontaktbasierten Verformung (z.B. zur Applanierung) des Auges 2 bei der Applikation der ophthalmologischen Vorrichtung 1 umfasst.

Die Laserquelle Q1 umfasst insbesondere einen Femtosekundenlaser zur Erzeugung eines gepulsten Laserstrahls L1 mit Femtosekundenlaserpulsen, die Pulsbreiten von typisch 10fs bis 1000fs (1fs=10⁻¹⁵s) aufweisen, im Infrarotbereich, beispielsweise bei einer Wellenlänge im Bereich von 1000nm bis 1200nm, z.B. 1053nm. Die Pulsrate der Femtosekundenlaserpulse liegt beispielsweise im Bereich von 10KHz bis 500KHz.

Die zweite Laserquelle Q2 umfasst einen Infrarotlaser zur Erzeugung eines zweiten (gepulsten) Laserstrahls L2 im mittelwelligen Infrarotbereich, vorzugsweise im Bereich von 2.9µm bis 3.0µm, beispielsweise einen Laser wie in der Patentanmeldung US 2002/0133146 beschrieben. Die Pulsrate des im mittelwelligen IR Bereich liegenden Laserstrahls liegt beispielsweise im Bereich von 50Hz bis 2KHz.

Vorzugsweise sind die Laserquellen Q1, Q2 in einem vom Lichtprojektor 11 getrennten Gehäuse 10 angeordnet. Die Laserquellen Q1, Q2 sind beispielsweise in einer Basisstation angeordnet, an welcher der Lichtprojektor 11 mittels einer Trägervorrichtung angebracht ist. Die Trägervorrichtung umfasst beispielsweise einen Spiegelgelenkarm.

In einem in der Figur 1 schematisch dargestellten ersten Betriebsmodus (zum Schneiden eines Gewebelappens, insbesondere eines Hornhautlappens) werden der gepulste Laserstrahl L1 und in einem in der Figur 2 schematisch dargestellten zweiten Betriebsmodus (zum Abtragen von Augengewebe, insbesondere Hornhautgewebe) der gepulste Laserstrahl L2 über das optische Übertragungssystem 12 dem Lichtprojektor 11 zugeführt. Der Lichtprojektor 11 projiziert den gepulsten Laserstrahl L1' respektive L2' auf eine durch die Fokalfläche definierte Bearbeitungsfläche, beispielsweise auf die durch die x/y-Koordinaten aufgespannte Fokalebene respektive Bearbeitungsebene.

Das optische Übertragungssystem 12 umfasst auch ein Scannersystem 15 zum Bewegen des Fokus F des Laserstrahls L1' respektive L2' auf der Bearbeitungsfläche. Das Scannersystem 15 umfasst Ablenkungselemente zum Ablenken der Laserstrahlen Q1, Q2 in der Vorschubrichtung x und/oder in der Abtastrichtung y, beispielsweise einen Galvanoscanner, einen resonanten Spiegelscanner, einen akustischen optischen Modulator (AOM), einen Polygonscanner oder einen mikroelektromechanischer Scanner (MEM). In einer Ausführungsvariante umfasst die ophthalmologische Vorrichtung 1 auch einen oder mehrere Bewegungstreiber 16 zum mechanischen Verschieben des Lichtprojektors 11 oder von Teilen des Lichtprojektors 11 in der Vorschubrichtung x und/oder in der Abtastrichtung y.

Die ophthalmologische Vorrichtung 1 umfasst zudem Fokussiermittel 14 zum variieren des Fokusdurchmessers ("spot size") des Fokus F. Im ersten Betriebsmodus werden die Femtosekundenlaserpulse des Laserstrahls L1' auf einen Fokusdurchmesser fokussiert, der' wesentlich kleiner ist, als der Fokusdurchmesser bei der fokussierten Projektion des Laserstrahls L2' im zweiten Betriebsmodus. Im ersten Betriebsmodus liegt der Fokusdurchmesser der Femtosekundenlaserpulse im Bereich von 0.5µm bis 10µm. Im zweiten Betriebsmodus liegt der Fokusdurchmesser des Laserstrahls L2' im Bereich von 100µm bis 500µm. Die Fokussiermittel 14 umfassen beispielsweise geeignete verschiebbare oder einstellbare Blenden und/oder bewegbare Linsen, welche durch das Steuermodul 13 entsprechend dem Betriebsmodus respektive dem erforderlichen Fokusdurchmesser angesteuert werden. Wie in den Figuren 1 und 2 schematisch dargestellt ist, sind die Fokussiermittel 14 im optischen Übertragungssystem 12 und/oder im Lichtprojektor 11 angeordnet.

Die Fokussiermittel 14 sind zudem eingerichtet und so ansteuerbar, dass sie sowohl im ersten Betriebsmodus die Femtosekundenlaserpulse des Laserstrahls L1', bei einem Arbeitsabstand Z₁ zwischen dem Lichtprojektor 11 und der Bearbeitungsfläche, auf die Bearbeitungsfläche im Innern des Augengewebes fokussieren, als auch im zweiten Betriebsmodus die Laserpulse des Laserstrahls L2', bei einem grösseren Arbeitsabstand Z₂ zwischen dem Lichtprojektor 11 und der Bearbeitungsfläche, auf die Bearbeitungsfläche auf dem freigelegten Augengewebe fokussieren. Zum Verschieben des Fokus F entlang der Projektionsachse z des Lichtprojektors 11, beispielsweise senkrecht zu der durch die Vorschubrichtung x und die Abtastrichtung y aufgespannten Bearbeitungsfläche, umfassen die Fokussiermittel 14 vorzugsweise eine bewegliche Fokussierungslinse und ein damit gekoppeltes Antriebselement. In einer alternativen Variante wird der Lichtprojektor 11 zum Verschieben des Fokus F mechanisch bewegt.

Zum Einstellen des Arbeitsabstands Z₁, Z₂ zwischen dem Lichtprojektor 11 und der Bearbeitungsfläche, d.h. zum Positionieren des Lichtprojektors 11 relativ zum Auge 2, umfasst die ophthalmologische Vorrichtung 1 je nach Ausführungsvariante verschiedene-Mittel zur Tiefenpositionierung, d.h. zum Positionieren des Lichtprojektors 11 entlang der Projektionsachse z. Der Arbeitsabstand z₁ für den ersten Betriebmodus wird vorzugsweise über Befestigungselemente zum Fixieren des Auges, z.B. einen Saugring, und Distanzelemente zwischen Auge 2 und Lichtprojektor 11 bestimmt, z.B. Kontaktelemente von definierter Dicke. Auf der Basis der bekannten mechanischen Abmessungen beim Arbeitsabstand Z₁ des Lichtprojektors 11 können die Fokussiermittel 14 für die Fokussierung der Femtosekundenlaserpulse auf die Bearbeitungsfläche im Innern des Augengewebes angesteuert werden.

Im zweiten Betriebmodus wird der Lichtprojektor 11 bei weggeklapptem Gewebelappen (Hornhautlappen) 22 zunächst mit dem Arbeitsabstand z₂ von der Oberfläche des abzutragenden Augengewebes positioniert, vorzugsweise mittels eines mechanischen Bewegungstreibers 17 oder durch manuelle Positionierung, beispielsweise mittels eines Gelenkarms. Beim Arbeitsabstand Z₂ des Lichtprojektors 11 werden die Fokussiermittel 14 basierend auf einer messtechnischen oder rechnerischen Ermittlung des Arbeitsabstands Z₂ zur Fokussierung der Laserpulse des Laserstrahls L2' auf die Bearbeitungsfläche angesteuert. Die messtechnische Ermittlung des Arbeitsabstands z₂ erfolgt beispielsweise mittels einer laserbasierten Distanzmessung. Die rechnerische Ermittlung des Arbeitsabstands Z₂ erfolgt beispielsweise auf der Basis der bekannten mechanischen Verschiebung des Lichtprojektors 11 vom vorherigen Arbeitsabstand Z₁ zum neuen Arbeitsabstand Z₂, wobei die (relative) mechanische Verschiebung beispielsweise direkt beim Bewegungstreiber 17 gemessen und an das Steuermodul 13 übermittelt wird. In einer Ausführungsvariante umfasst die ophthalmologische Vorrichtung 1 zudem ein nicht dargestelltes Augenüberwachungsmodul (ein so genannter "Eye Tracker") zur Bestimmung von Augenbewegungen.

In einer weiteren Ausführungsvariante ist das Steuermodul 13 eingerichtet, in einem-dritten Betriebsmodus die Laserquelle Q1 und/oder die Fokussiermittel 14 derart anzusteuern, dass der Laserstrahl L1 in einem dritten Betriebsmodus mit reduzierter Intensität in das Augengewebe eingestrahlt wird, so dass die Augenhornhaut 21 durch das Licht im kurzwelligen IR Bereich des Femtosekundenlasers ausgeleuchtet wird, ohne dass sie beschädigt wird. Vorzugsweise werden die Fokussiermittel 14 durch das Steuermodul 13 derart angesteuert, dass sie in einen Zustand gesetzt werden, in welchem sie den Fokusdurchmesser derart aufweiten, dass die Intensität nicht ausreicht um das Augengewebe zu beeinträchtigen. In einer Variante wird die Laserquelle Q1 durch das Steuermodul 13 zusätzlich oder alternativ so angesteuert, dass sie die Intensität des Laserstrahls L1 reduziert.

Das Steuermodul 13 ist vorzugsweise als programmiertes Logikmodul mittels Software und/oder Hardware ausgeführt. Das Steuermodul 13 ist zur Übertragung von Steuersignalen, Steuerdaten und/oder Messdaten mit dem Scannersystem 15, den Fokussiermitteln 14, den Lasserquellen Q1, Q2 und/oder den Bewegungstreibern 16, 17 verbunden. Das Steuermodul 13 ist in einem separaten oder in einem mit dem Lichtprojektor 11 gemeinsamen Gehäuse angeordnet.

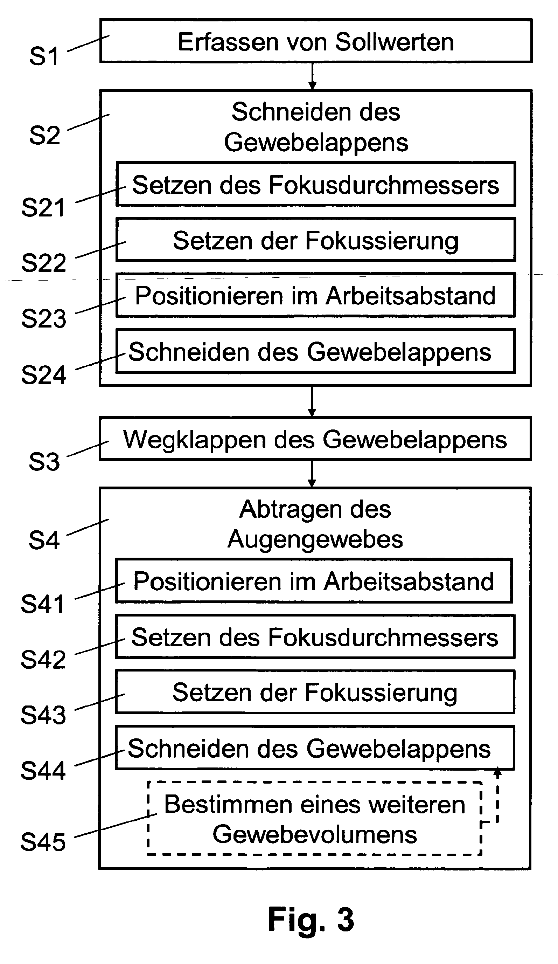

In den nachfolgenden Abschnitten wird mit Bezug zu der Figur 3 der durch das Steuermodul 13 gesteuerte Ablauf beim Schneiden eines Gewebelappens 22 im ersten Betriebsmodus und bei der Gewebeabtragung zur refraktiven Korrektur des Auges 2 im zweiten Betriebsmodus beschrieben.

Im Schritt S1 bestimmt das Steuermodul 13 die Sollwerte für chirurgische Behandlung des Auges 2. Die Sollwerte werden beispielsweise über eine Benutzerschnittstelle eingegeben und im Steuermodul 13 erfasst. Es werden Sollwerte für die gewünschte refraktive Korrektur des Auges 2 und optional die gewünschte Grösse (Durchmesser) und Tiefe des zu schneidenden Gewebelappens 22 erfasst.

Im Schritt S2 setzt das Steuermodul 13 die ophthalmologische Vorrichtung 1 in den ersten Betriebsmodus und führt in diesem Modus die Schritte zum Schneiden des Gewebelappens 22 aus.

Im Schritt S21 steuert das Steuermodul 13 die Fokussiermittel 14 so, dass die Fokussiermittel 14 in einen Zustand versetzt werden, in welchem der Laserstrahl L1' mit den Femtosekundenlaserpulsen, wie oben angeführt, zum Ausführen eines präzisen Schnitts im Augengewebe (Augenhomhaut 21) stark fokussiert wird, und der Fokusdurchmessers des Fokus F im Bereich von 0.5µm bis 10µm liegt.

Im Schritt S22 steuert das Steuermodul 13 die Fokussiermittel 14 so, dass die Fokussiermittel 14 in einen Zustand versetzt werden, in welchem der Laserstrahl L1' mit den Femtosekundenlaserpulsen im Arbeitsabstand Z₁ des Lichtprojektors 11 fokussiert auf die Bearbeitungsfläche projiziert wird, wo der Schnitt im Augengewebe auszuführen ist. Die Schritte S21 und S22 können auch in umgekehrter Reihenfolge oder zusammen ausgeführt werden.

Im Schritt S23 wird der Lichtprojektor 11 im Arbeitsabstand Z₁ zur Bearbeitungsfläche positioniert. Der Schritt S23 kann auch schon früher, beispielsweise vor den Schritten S21 und S22 erfolgen.

Im Schritt S24 aktiviert das Steuermodul 13 die Laserquelle Q1 und aktiviert oder steuert das Scannersystem 15 so, dass der Fokus F des Laserstrahls L1' auf der Bearbeitungsfläche im Augengewebe gemäss einem Abtastmuster zum Schneiden des Gewebelappens 22 bewegt wird. Nach Beendigung des Schnitts endet der erste Betriebsmodus und das Steuermodul zeigt dies dem Benutzer optisch und/oder akustisch an.

Im Schritt S3 wird der Gewebelappen 22, wie in der Figur 2 schematisch dargestellt, weggeklappt.

Im Schritt S4 setzt das Steuermodul 13 die ophthalmologische Vorrichtung 1 in den zweiten Betriebsmodus und führt in diesem Modus die Schritte zum Gewebeabtragen für die refraktive Korrektur aus.

Im Schritt S41 wird der Lichtprojektor 11 im Arbeitsabstand Z₂ zur Bearbeitungsfläche positioniert. Bei einer manuellen Positionierung, nimmt das Steuermodul vom Benutzer beispielsweise über Bedienungselemente ein Steuersignal entgegen, das anzeigt, dass der Lichtprojektor 11 in den gewünschten Arbeitsabstand z₂ positioniert wurde. Das Steuermodul ermittelt den Wert des Arbeitsabstands z₂, wie oben beschrieben, beispielsweise messtechnisch oder rechnerisch.

Im Schritt S42 steuert das Steuermodul 13 die Fokussiermittel 14 so, dass die Fokussiermittel 14 in einen Zustand versetzt werden, in welchem der Laserstrahl L2', zum Abtragen (Auflösen) von Augengewebe im Unterschied zum vorherigen (internen) Gewebeschneiden auf der durch Wegklappen des Gewebelappens freigelegten Bearbeitungsfläche weniger stark fokussiert wird, und der Fokusdurchmessers des Fokus F im Bereich von 100µm bis 500µm liegt.

Im Schritt S43 steuert das Steuermodul 13 die Fokussiermittel 14 so, dass die Fokussiermittel 14 in einen Zustand versetzt werden, in welchem der Laserstrahl L2' im Arbeitsabstand z₂ des Lichtprojektors 11 fokussiert auf die Bearbeitungsfläche projiziert wird. Die Schritte S42 und S43 können auch in umgekehrter Reihenfolge oder zusammen ausgeführt werden.

Im Schritt S44 aktiviert das Steuermodul 13 die Laserquelle Q2 und aktiviert oder steuert das Scannersystem 15 so, dass der Fokus F des Laserstrahls L2' auf der freigelegten Bearbeitungsfläche für den Gewebeabtrag zur gewünschten refraktiven Korrektur bewegt wird. Das entsprechende abzutragende Gewebevolumen wird dabei beispielsweise auf Grund einer Tabelle bestimmt, in weicher unterschiedliche Gewebevolumen, z.B durch örtliche Verteilung und/oder Abtastmuster für Fokus F definiert, unterschiedlichen Werten von refraktiven Korrekturen zugeordnet sind. Bei der Positionierung des Fokus F berücksichtigt das Scannersystem 15 respektive das Steuermodul 13 in einer Variante die vom Augenüberwachungsmodul detektierten Augenbewegungen. Nach Beendigung des Gewebeabtrags endet der zweite Betriebsmodus und das Steuermodul zeigt dies dem Benutzer optisch und/oder akustisch an.

Im optionalen Schritt S45 wird beispielsweise messtechnisch bestimmt, ob die gewünschte refraktive Korrektur erreicht wurde. Falls die gewünschte Korrektur erreicht wurde, beendet das Steuermodul 13 das Verfahren. Andernfalls, wenn die gewünschte refraktive Korrektur noch nicht erreicht wurde, bestimmt das Steuermodul ein zusätzliches Gewebevolumen, das zum Erreichen der gewünschten refraktiven Korrektur abzutragen ist und die Behandlung fährt erneut im Schritt S44 weiter.

## Patentansprüche

1. Ophthalmologische Vorrichtung (1) für eine refraktive Korrektur eines Auges (2), umfassend:
eine erste Laserquelle (Q1) zur Erzeugung eines gepulsten ersten Laserstrahls (L1) mit Femtosekundenlaserpulsen,
eine zweite Laserquelle (Q2) zur Erzeugung eines zweiten Laserstrahls (L2) im mittelwelligen Infrarotbereich, und
einen gemeinsamen optischen Lichtprojektor (11) zur fokussierten Projektion des ersten Laserstrahls (L1) in einem ersten Betriebsmodus und zur fokussierten Projektion des zweiten Laserstrahls (L2) in einem zweiten Betriebsmodus jeweils zum Auflösen von Augengewebe.

2. Vorrichtung (1) nach Anspruch 1, **gekennzeichnet durch** Fokussiermittel (14), welche eingerichtet sind zum Fokussieren der Projektion des ersten Laserstrahls (L1) im ersten Betriebsmodus mit einem ersten Fokusdurchmesser und zum Fokussieren der Projektion des zweiten Laserstrahls (L2) im zweiten Betriebsmodus mit einem hinsichtlich des ersten Fokusdurchmessers um ein Mehrfaches grösseren zweiten Fokusdurchmesser.

3. Vorrichtung (1) nach Anspruch 2, **dadurch gekennzeichnet, dass** der erste Fokusdurchmesser im Bereich von 0.5µm bis 10µm liegt, und dass der zweite Fokusdurchmesser im Bereich von 100µm bis 500µm liegt.

4. Vorrichtung (1) nach einem der Ansprüche 1 bis 3, **gekennzeichnet durch** Mittel, welche eingerichtet sind den ersten Laserstrahl (L1) in einem dritten Betriebsmodus mit einer hinsichtlich der Projektion des ersten Laserstrahls (L1) im ersten Betriebsmodus und der Projektion des zweiten Laserstrahls (L2) im zweiten Betriebsmodus derart reduzierten Intensität in das Augengewebe zu projizieren, dass kein Augengewebe aufgelöst wird.

5. Vorrichtung (1) nach einem der Ansprüche 1 bis 4, **gekennzeichnet durch** Mittel zur Tiefenpositionierung des optischen Lichtprojektors (11) mit einem ersten Arbeitsabstand (z₁) zu einer Bearbeitungsfläche im Auge (2) im ersten Betriebsmodus und zur Tiefenpositionierung des optischen Lichtprojektors (11) mit einem zweiten Arbeitsabstand (z₂) zu der Bearbeitungsfläche im zweiten-Betriebsmodus, und-durch Fokussiermittel (14) zum Fokussieren der Projektion des ersten Laserstrahls (L1) im ersten Betriebsmodus auf die Bearbeitungsfläche zum Schneiden eines Gewebelappens (22), und zum Fokussieren der Projektion des zweiten Laserstrahls (L2) im zweiten Betriebsmodus auf die Bearbeitungsfläche zum Abtragen von Augengewebe bei weggeklapptem Gewebelappen (22).

6. Vorrichtung (1) nach einem der Ansprüche 1 bis 5, **dadurch gekennzeichnet, dass** die erste Laserquelle (Q1) eingerichtet ist, den ersten Laserstrahl (L1) mit einer Pulsrate zu erzeugen, die ein Mehrfaches der Pulsrate ist, welche die zweite Laserquelle (Q2) für den zweiten Laserstrahl (L2) erzeugt.

7. Vorrichtung (1) nach einem der Ansprüche 1 bis 5, **gekennzeichnet durch** ein Scannersystem (15) zur Fokusbewegung bei der fokussierten Projektion des ersten Laserstrahls (L1) und bei der fokussierten Projektion des zweiten Laserstrahls (L2).
